# EUROPEAN PATENT SPECIFICATION

(11) **EP 0 331 712 B1**
(45) Date of publication and mention of the grant of the patent: **18.05.1994**
(21) Application number: 88908590.8
(22) Date of filing: 31.08.1988
(51) Int. Cl.: A61F 5/04, F16B 15/06

(54) **SURGICAL FASTENER**
CHIRURGISCHES BEFESTIGUNGSMITTEL
DISPOSITIF DE FIXATION CHIRURGICAL

(30) Priority: 02.09.1987 US 92121
(43) Date of publication of application: 13.09.1989
(73) Proprietor: Warren, Russell F., Greenwich Connecticut 06831 (US)
(72) Inventor: Warren, Russell F., Greenwich Connecticut 06831 (US)
(74) Representative: Thomson, Paul Anthony
(86) International application number: US8803041
(87) International publication number: WO8901767

(56) References cited:
- EP-A- 0 238 223
- EP-A- 0 260 970
- DE-U- 8 633 339
- FR-A- 2 590 792
- US-A- 2 414 882
- US-A- 2 570 465
- US-A- 2 815 934
- US-A- 4 586 502
- US-A- 4 646 741
- US-A- 4 728 570
- US-A- 8 504 568

## Description

This invention relates to surgical devices in general, and more particularly to surgical fasteners of the sort adapted to attach soft tissues (e.g. ligaments, tendons and the like) to bone and bone-like structures.

Numerous surgical fasteners have been developed for use in joining together two or more body parts and/or prosthetic devices. See, for example, U.S. Patents Nos. 3716058 (Tanner, Jr.), 4060089 (Noiles), 4263903 (Griggs), 4454875 (Pratt et al.), 4548202 (Duncan), 4570623 (Ellison et al.), 4580563 (Gross), 4590928 (Hunt et al.), and 4635637 (Schreiber), and the references cited therein, and PCT Publication No. WO 85/03857 (Schreiber).

Still other fasteners have been developed for use in joining together two or more parts. See, for example, U.S. Patents Nos, 2523239 (Tinnerman), 2853913 (Rapata), 2927497 (Rapata), 3494244 (Wayland), 3810279 (Swick et al.), 4395174 (Freeman), 4396329 (Wollar), 4402641 (Arff), 4427328 (Kojima), and 4551189 (Peterson), and the references cited therein, and UK Patent Specification No. 520169 (Universal Rubber Paviors Limited) and Canadian Patent No. 1015989 (Russo).

International Published Application No. WO-A-8504568 described a surgical fastener for attaching a soft tissue to a bone, said fastener having a threaded shank to enable said fastener to be utilised as a screw, said shank having first and second ends, a longitudinal axis and at least one rib extending outwardly of the shank intermediate the first and second ends thereof, said shank also has an enlarged head disposed on the second end thereof. It is further disclosed that such fastener may be inserted by utilisation of a pilot opening.

US-A-2414882 and US-A-2570465 describe a bore extending along the longitudinal axis of a surgical screw.

It is known to make surgical fasteners of either absorbable or non-absorbable materials (e.g. US-A-4586502) and it is also known to adapt surgical ribs surrounding a shank in a drive fastener to engage apertures in a retaining plate (e.g. US-A-4646741).

It is an object of the present invention, based on the disclosure in WO-A-8504568, to provide a surgical fastener whereby bone necrosis is minimised. Furthermore, it is an object of the present invention to provide a fastener which is designed to withstand the shear and axial forces applied by ligaments, tendons and the like attached by the fastener to the bone. Furthermore, it is an additional object of the invention to provide a fastener having designed and spaced ribs thereon which provide the fastener with improved holding power.

According to the present invention there is provided an apparatus for attaching soft tissues to bone of the type comprising a surgical fastener adapted to directly engage a hole in the bone which includes a shank having a distal end, a proximal end, a longitudinal axis and at least one projection extending outwardly of the shank intermediate the distal and proximal ends thereof, and an enlarged head coupled to the proximal end of the shank, characterised in that:
(1) the distal end of the shank has a frusto-conical configuration; and
(2) the at least one projection comprises at least two discrete, substantially identical ribs, each rib (a) completely encircling the shank transversely to the longitudinal axis, (b) being longitudinally spaced from adjacent ones of said at least two ribs so as to promote bone ingrowth between adjacent ones of the ribs, (c) including a first longitudinal portion and a second longitudinal portion, the first longitudinal portion being disposed toward the distal end of the shank, the second longitudinal portion being disposed toward the proximal end of the shank and the first longitudinal portion having a smaller circumference than the second longitudinal portion, (d) a bore (165) extending completely through the shank and the head along the longitudinal axis, and (e) delivery means for delivering the fastener to the attachment point of the fastener to the bone.

The foregoing fastener is used to attach soft tissues (e.g. ligaments, tendons and the like) to bone and bone-like structures in the following manner. First, the soft tissue is placed against the bone and then a thin guide wire is passed through the soft tissue and into the bone. A cannulated drill is then loaded coaxially onto the guide wire and is moved down the guide wire and worked on the guide wire so as to form a hole through the soft tissue and into the bone. Next, the cannulated drill is withdrawn from the guide wire and the aforementioned surgical fastener is loaded coaxially onto the guide wire. Then a hollow driver is loaded coaxially onto the guide wire and is used to successively strike the head of the fastener so as to drive the shank of the fastener through the soft tissue and into the bone, with the head of the fastener engaging the soft tissue and captivating it against the bone. Thereafter, the hollow driver is withdrawn from the guide wire, and then the guide wire is removed from the soft tissue and the bone, leaving the soft tissue securely attached to the bone by the surgical fastener.

### Brief Description Of The Drawings

These and other objects and features of the present invention will be more fully disclosed or rendered obvious in the following detailed description of the invention, which is to be considered together with the accompanying drawings wherein like numbers refer to like parts and further wherein:
Fig. 1 is a side elevation of a surgical fastener made in accordance with the present invention;
Fig. 2 is a top view of the same surgical fastener, taken along line 2-2 of Fig. 1;
Fig. 3 is a bottom view of the same surgical fastener, taken along line 3-3 of Fig. 1;
Fig. 4 is a partial side elevation showing a soft tissue (e.g. a ligament, tendon or the like) placed against a bone, with a guide wire having been inserted through the soft tissue and into the bone;
Fig. 5 is a view like that of Fig. 4, except that a cannulated drill has been loaded coaxially onto the guide wire and used to drill through the soft tissue and into the bone;
Fig. 6 is a view like that of Fig. 5, except that the cannulated drill has been removed from the guide wire and a surgical fastener of the type shown in Fig. 1 has been loaded coaxially onto the guide wire, and a hollow driver has been loaded coaxially onto the guide wire;
Fig. 7 is a view like that of Fig. 6, except that the fastener has been fully driven through the soft tissue and into the bone by the hollow driver so as to securely fasten the soft tissue to the bone;
Fig. 8 is a view like that of Fig. 7, except that the hollow driver and the guide wire have been removed;
Fig. 9 is a view like that of Fig. 1, except that an alternative form of surgical fastener having eight ribs is shown;
Fig 10. is a top view of the surgical fastener shown in Fig. 9, taken along line 10-10 of Fig. 9;
Fig. 11 is a bottom view of the surgical fastener shown in Fig. 9, taken along line 11-11 of Fig. 9; and
Fig. 12 is a view like that of Fig. 1, except that another alternative form of surgical fastener having a modified rib construction is shown.

### Detailed Description Of The Invention

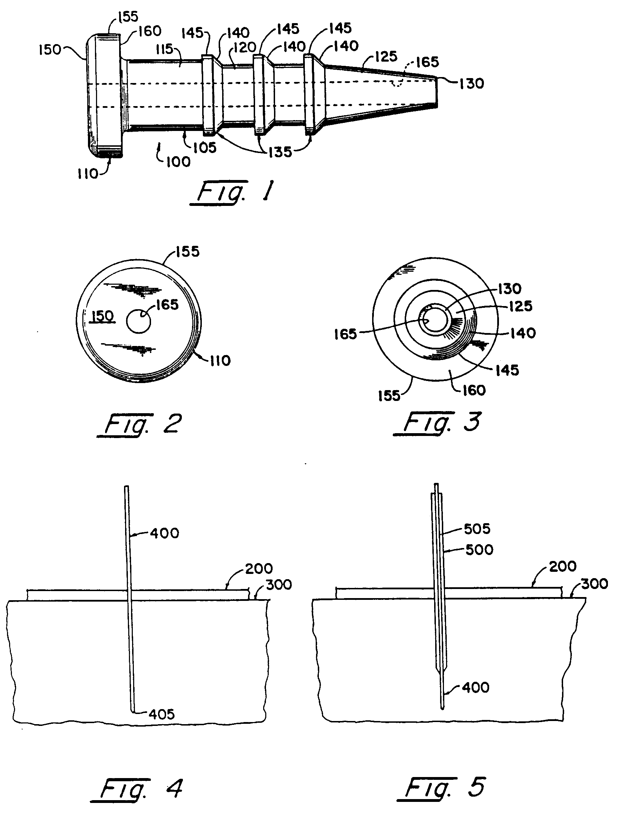

Looking first at Figs. 1-3, there is shown a surgical fastener 100 made in accordance with the present invention. Fastener 100 comprises a shank 105 and a head 110 which are preferably formed integral with one another. Shank 105 comprises a first portion 115 which has an outer diameter of approximately 0.35cm (0.138 inches), a second portion 120 which has an outer diameter of approximately 0.30cm (0.118 inches), and a third portion 125 which has an outer diameter of approximately 0.30cm (0.118 inches) adjacent second portion 120 and which narrows (at approximately an 8-14 degree taper) to a diameter of approximately 0.16cm (0.063 inches) immediately adjacent the fastener's front tip surface 130.

Shank 105 has three radially projecting ribs 135 disposed along its length. One of the ribs 135 is disposed at the intersection of first portion 115 and second portion 120, one of the ribs 135 is disposed at the intersection of second portion 120 and third portion 125, and one of the ribs is disposed intermediate the length of second portion 120. Ribs 135 each comprise a leading surface 140 set at an approximately 45 degree angle to the longitudinal axis of fastener 100, and a peripheral surface 145 which extends approximately 0.05cm (0.020 inches) along the longitudinal axis of fastener 100 and which has an outer diameter of approximately 0.40cm (0.157 inches).

Head 110 has an outer diameter of approximately 0.64cm (0.256) inches and terminates in a top end surface 150, a cylindrical side surface 155 and a lower surface 160. Head 110 has a thickness of approximately 0.174cm (0.069 inches), when measured from its top end surface 150 to its lower surface 160.

Surgical fastener 100 has an overall length of approximately 1.40cm (0.710 inches), when measured from its front tip surface 130 to its top (i.e., trailing) end surface 150.

A bore 165 having an internal diameter of approximately 0.122cm (0.048 inches) passes completely through the fastener along its longitudinal axis, i.e., it extends from the fastener's front tip surface 130 to its top (i.e., trailing) end surface 150.

Looking next at Figs. 4-8, the aforementioned surgical fastener 100 is intended to be used to attach a soft tissue (e.g. a ligament, tendon or the like) 200 to a bone (or another bone-like structure) 300.

For the sake of convenience, soft tissue 200 will hereinafter be described as a ligament 200; however, it is to be understood that member 200 might also constitute a tendon or some other type of soft tissue which is to be attached to a bone (or another bone-like structure) 300.

Looking specifically now at Fig. 4, ligament 200 is first positioned against the bone 300 which it is to be attached to. Next, a long thin guide wire 400 is passed through ligament 200 and into bone 300. Guide wire 400 has a diameter of approximately 0.089cm (0.035 inches) and its leading tip 405 preferably penetrates bone 300 to a depth of at least 1.91cm (0.75 inches). It is to be appreciated that the guide wire's leading tip 405 is substantially pointed so that it can more easily penetrate through ligament 200 and into bone 300. Guide wire 400 may be emplaced simply by pushing it through the ligament and into the bone, or it may be mounted in a drilling device (not shown) to facilitate entry. In the event that the guide wire 400 is intended to be simply pushed through ligament 200 and into bone 300, a supporting cannula of the sort well known in the art (not shown) may be concentrically mounted around at least a portion of the guide wire during insertion so as to help maintain the linear shape of the guide wire during penetration. If such a supporting cannula is used, it is removed from around the guide wire as soon as the guide wire has been properly positioned in the manner shown in Fig. 4. Alternatively, in the event that guide wire 400 is intended to be drilled through ligament 200 and into bone 300, the guide wire's leading tip 405 may also include a helical drilling thread (not shown) to facilitate penetration. If a drilling device is used to deploy guide wire 400, the drilling device is detached from the guide wire as soon as the guide wire has been properly positioned in the manner shown in Fig. 4.

Looking next at Fig. 5, a cannulated drill 500 having an axial bore 505 is then concentrically mounted onto the free top end of guide wire 400 and is moved downward along the guide wire until the drill's leading tip contacts the upper surface of ligament 200. Then, using guide wire 400 as a drilling guide, cannulated drill 500 is drilled through ligament 200 and into bone 300. Cannulated drill 505 is sized so as to form a hole approximately 0.359cm (0.134 inches) in diameter in ligament 200 and bone 300. Cannulated drill 500 cuts into bone 300 to a depth of approximately 1.91cm (0.75 inches), whereupon it is withdrawn from ligament 200 and bone 300 and then removed from the guide wire. It is to be appreciated that the entire time the foregoing is being accomplished, guide wire 400 is left in place in bone 300.

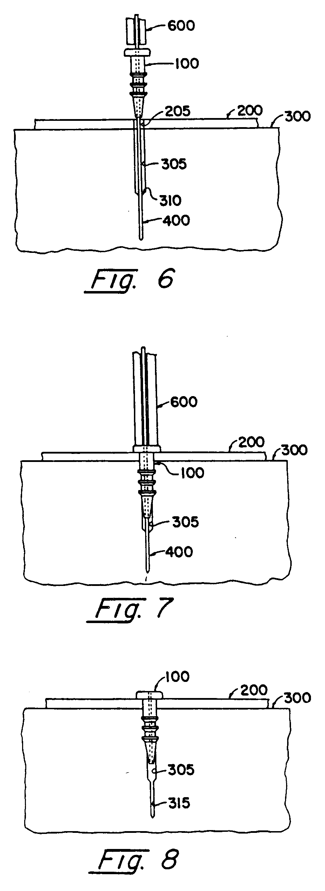

It is also to be appreciated that when cannulated drill 500 is removed from guide wire 400, a hole will be left in ligament 200 and bone 300, with guide wire 400 being disposed axially within the hole. As seen in Fig. 6, the portion of the hole extending through ligament 200 (indicated generally at 205) may tend to close somewhat upon the withdrawal of cannulated drill 500 due to the somewhat resilient nature of ligament 200, whereas the portion of the hole extending into bone 300 (indicated generally at 305) may not. The hole portion 305 will tend to terminate in a bone shoulder 310 at the base of the hole.

Looking now at Fig. 6, the surgical fastener 100 is then loaded concentrically onto guide wire 400, with the fastener's front tip surface 130 leading, so that the guide wire passes through the fastener's central bore 165. Depending on the tightness of the fit between fastener 100 and guide wire 400, as well as the presence of any intervening obstacles, fastener 100 may then slide down guide wire 400 under the influence of gravity until the fastener contacts ligament 200, or it may simply sit at the top end of guide wire 400, waiting to be urged down the guide wire. In this respect, it is to be appreciated that even if fastener 100 should migrate down guide wire 400 on its own, inasmuch as the hole 205, 305 formed in ligament 200 and bone 300 has a diameter of approximately 0.359cm (0.134 inches), and inasmuch as ligament 200 may also tend to close somewhat about the hole portion 205 as noted above, and also inasmuch as the ribs 135 of fastener 100 have an outer diameter of approximately 0.157 inches, the fastener will in any case tend to engage and be stopped by the ligament.

Still looking at Fig. 6, a hollow driver 600 is then coaxially fitted onto the free top end of guide wire 400. Hollow driver 600 has an internal bore diameter of approximately 0.140cm (0.055 inches) and an external diameter of approximately 0.635cm (0.25 inches), whereby hollow driver 600 can move easily up and down guide wire 400 to contact the head of fastener 100.

Looking next at Fig. 7, hollow driver 600 is then used to first move fastener 100 down the guide wire to engage ligament 200 if the fastener is not yet in engagement with the ligament, and then to repeatedly strike fastener 100 on its upper surface 150, whereby the fastener's shank can be driven through ligament 200 and into bone 300 so that the shank locks itself into bone 300 while the head of the fastener captivates the ligament against the bone. It is to be appreciated that ribs 135 are sized and formed so that they strongly engage, and form a good grip with, the walls of bone 300 defining bore portion 305.

Finally, once the fastener has been completely driven into bone 300 so that the head of the fastener securely captivates the ligament against the bone, hollow driver 600 is withdrawn from it coaxial position on guide wire 400, and guide wire 400 is then withdrawn from fastener 100, ligament 200 and bone 300, leaving fastener 100, ligament 200 and bone 300 securely disposed in the positions shown in Fig. 8. It is to be appreciated that upon the removal of guide wire 400 from bone 300, an additional bore section 315 (corresponding to the location of guide wire 400 prior to its removal) may remain in bone 300 below bore section 305. It is also to be appreciated that upon the removal of guide wire 400 from bone 300, the fastener's central bore 165 may then be sealed by ways well known in the art (e.g. by filling with bone wax) so as to seal off the interior of bone 300 from the region outside the bone.

In some circumstances it may be desired to have fastener 100 remain permanently in the body, without any substantial degradation over time; in this event, fastener 100 may be formed out of materials such as polypropylene, Hytrel (R) copolyester, or homopolymer polyesters, e.g. polyethylene terephthalate or polybutylene terephthalate, or nylon, or polyethylene, or polycarbonate, or acrylonitrile butadiene styrene block copolymers.

In other circumstances it may be desired to have fastener 100 remain intact in the body only temporarily, and to have it thereafter degrade and be naturally absorbed into the body after the passage of some time interval; in this event, fastener 100 may be formed out of materials such as homo and copolymers of glycolide and trimethylene carbonate, homo and copolymers of lactide and glycolide, or a blend of these homopolymers and copolymers. Such fasteners may also be coated with longer lasting materials, e.g. caprolactone and glycolide homo and copolymers, or glycolide and lactide homo and copolymers. Of course, the exact composition of such absorbable fasteners will vary according to the absorption characteristics desired. Such compositions are well known to persons skilled in the art.

The fasteners may also be made out of composites of the foregoing nonabsorbable and absorbable materials and bone growth promoters, e.g. hydroxyapatite, calcium phosphate, etc., or other ceramics.

The fasteners may also be coated with functional agents such as antibiotics, anticancer drugs, etc. to facilitate delivery of the agents to the implant site.

If possible, fastener 100 is preferably formed out of a slightly resilient material, whereby the fastener's ribs 135 may deform slightly upon engagement with ligament 200 and especially bone 300 and thereafter "spring back" to form a good grip with the walls of bone 300 defining bore portion 305. Also, if possible, fastener 100 is formed out of a material known to minimize bone necrosis.

It should be noted that certain features of fastener 100 facilitate its deployment and function. For example, the narrowing character of the fastener's shank portion 125 provides a somewhat pointed front end which helps guide the insertion of fastener 100 into ligament 200 and bone 300. Also, the enlarged diameter of the fastener's shank portion 115 (relative to its shank portion 120) provides added strength to help keep the deployed fastener intact when it is subsequently subjected to stresses in the patient's body. Also, the fastener's ribs 135 are positioned along shank 105 so that they preferably reside inside the bone's interior, softer cancellous region, rather that residing inside the bone's more brittle exterior cortical region. Furthermore, the intersection of the fastener's top end surface 150 and its cylindrical side surface 155 is rounded slightly so as to minimize any interference by the fastener with other bodily parts.

It should also be noted that the features of fastener 100 and the specific manner in which it is intended to be deployed facilitate the use of the fastener in arthroscopic applications wherein the fastener must generally be deployed in a remote location which can be reached only through a narrow cannula providing an access portal of a few millimeters.

It is also to be appreciated that certain changes could be made to the surgical fastener 100 described above without departing from the scope of the present invention.

Thus, for example, the overall appearance of fastener 100 could be maintained but its dimensions changed, e.g. the fastener could be enlarged so that it has a length of 3.60cm (1.420 inches) (rather than 1.40cm (0.710 inches) as described above), with the various dimensions of the fastener being correspondingly enlarged, or the fastener could be reduced so that it has a length of 0.91cm (0.36 inches) (rather than 1.40cm (0.710 inches) as describe above), with the various dimensions of the fastener being correspondingly reduced. Alternatively, the dimensions of the fastener could be changed so as to make the fastener longer and thinner, or shorter and fatter, etc. In this regard, it is to be noted that the dimensions of the surgical fastener 100 given above are believed to be optimum for use in attaching ligaments to bones in the shoulder region; however, for other purposes (e.g., for attaching ligaments to bones in the leg region), other dimensions may be more desirable. It is also to be appreciated that as the thickness of fastener 100 is altered, the size of the bore 205, 305 formed in ligament 200 and bone 300 may also need to be correspondingly adjusted so as to enable fastener 100 to properly engage ligament 200 and bone 300 when the fastener is deployed.

It is also anticipated that the thickness of guide wire 400 may also be varied, e.g. guide wire 400 might be increased in diameter to a thickness of approximately 0.15cm (0.06 inches), or guide wire 400 might be reduced in diameter to a thickness of approximately 0.064cm (0.025 inches). Of course, in the event that the thickness of guide wire 400 is changed, the size of the bore 165 in fastener 100 and the size of the internal bores in cannulated drill 500 and hollow driver 600 may also need to be correspondingly changed.

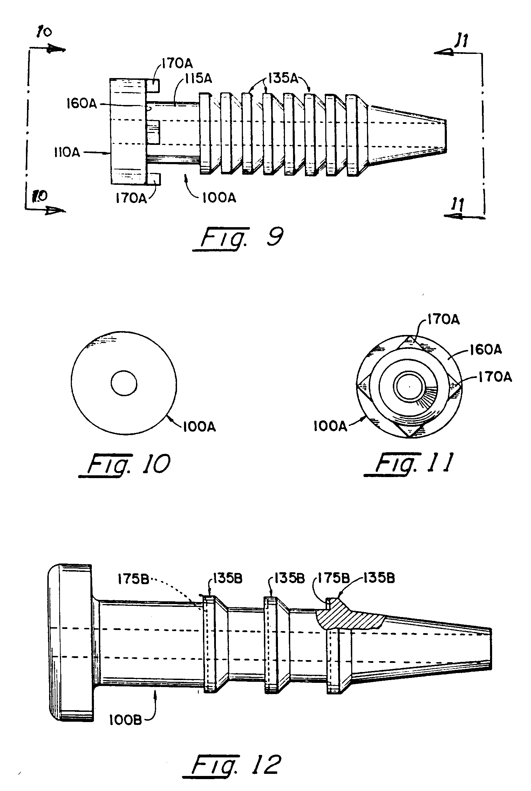

It is also anticipated that surgical fastener 100 might be formed with more or less ribs 135 than the three ribs shown in Figs. 1-8. Thus, for example, a surgical fastener 100A having eight ribs 135A is shown in Figs. 9-11. Also, as seen with fastener 100A of Figs. 9-11, the ribs may contact one another directly (rather than being spaced from one another as is the case with fastener 100 shown in Figs. 1-8), and the fastener's shank portion 115A may not be enlarged relative to the remainder of the fastener's shank. Furthermore, it is anticipated that a plurality of ligament-engaging projections 170A could be added to the lower surface 160A of head 110A to enhance the engagement of the fastener's head with ligament 200.

It is also anticipated that the fastener's ribs 135 might be undercut slightly at their top sides so as to make them slightly more flexible. Thus, for example, a fastener 100B having undercuts as shown at 175B in each of its ribs 135B is shown in Fig. 12.

It is also to be appreciated that while the foregoing surgical fastener was described in the context of attaching a ligament to a bone, it might also be used to attach other ligament-like objects (e.g. a tendon or some other type of soft tissue), both natural and man-made, to bone, or it might be used to attach ligaments or ligament-like objects to bone-like structures, i.e., the fastener might be used to attach an artificial ligament to a natural bone, or a natural ligament to an artificial prosthesis, etc.

Furthermore, it is also to be appreciated that certain changes might be made in the manner in which the surgical fastener is deployed. Thus, for example, whereas in the foregoing description guide wire 400 is first set and cannulated drill 500 is then coaxially mounted onto the guide wire and then drilled into ligament 200 and bone 300, it is anticipated that the sequence could be altered somewhat so that cannulated drill 500 is first drilled into ligament 200 and bone 300, then while cannulated drill 500 is positioned in ligament 200 and bone 300, guide wire 400 is passed through the cannulated drill's central bore 505 to engage bone 300, and then cannulated drill 500 is withdrawn, leaving guide wire 400 in place in ligament 200 and bone 300. This alternative arrangement may be advantageous in the sense that cannulated drill 500 can act as a supporting cannula to help keep the thin guide wire straight during insertion, although it does have the disadvantage that the guide wire is not present during drilling by cannulated drill 500 so as to act as a guide for the drill.

These and other changes of their type are considered to be within the scope of the present invention.

### Examples

The following examples are provided to illustrate the fabrication of the novel fastener herein disclosed.

### Example 1:

The 3 rib fastener of Figs. 1-8 was injection molded out of Maxon (R) (a copolymer comprising trimethylene carbonate and glycolide). The fastener had the following dimensions:

| | |
|---|---|
| (1) Rib Diameter | 0.40cm (0.157 inches) |
| (2) Head Diameter | 0.64cm (0.256 inches) |
| (3) Shaft Diameter | 0.35cm (0.138 inches) |
| (4) Overall Length | 1.40cm (0.710 inches) |
| (5) Center Bore Diameter | 0.122cm (0.048 inches) |

The fasteners were post treated at 110 degrees centigrade for 16 hours, and then ETO (ethylene oxide) sterilized. The fasteners were then implanted in canine humeri and demonstrated to work in a satisfactory manner.

### Example 2:

The 3 rib fastener was molded out of Lactide/Glycolide copolymer (80/20 composition) for the nominal dimensions shown in Example 1 above. The fasteners were tested in canine humeri without any post treatment, and were demonstrated to work in a satisfactory manner.

### Example 3:

The 8 rib fastener of Figs. 9-11 was molded out of Maxon (see above) and was then coated by dipping in an approximately 10% solution of polylactic acid in methylene chloride. The fasteners were then tested and were found to work in a satisfactory manner.

### Example 4:

Hydroxyapatite powder (Calcitech, San Diego, CA) was dry blended with Maxon (see above) and the 3 rib fasteners of Figs. 1-8 were molded, at an initial loading of 10% hydroxyapatite (HA) by weight. The fasteners were then tested and were found to work in a satisfactory manner.

### Advantages Of The Invention

Numerous advantages are achieved by using the present invention.

First, the present invention provides a novel surgical fastener of the sort adapted to attach soft tissues (e.g. ligaments, tendons and the like) to bone and bone-like structures.

Second, the present invention provides a novel surgical fastener of the sort adapted to attach soft tissues (e.g. ligaments, tendons and the like) to bone and bone-like structures, wherein the fastener is designed to minimize bone necrosis.

Third, the present invention provides a novel surgical fastener of the sort adapted to attach soft tissues (e.g. ligaments, tendons and the like) to bone and bone-like structures, wherein the fastener is designed to be used arthroscopically.

Fourth, the present invention provides novel means for deploying the aforementioned surgical fastener in a remote, e.g. arthroscopic, location.

Fifth, the present invention provides a complete system for attaching soft tissues (e.g., ligaments, tendons and the like) to bone and bone-like structures.

And sixth, the present invention provides a novel method for attaching soft tissues (e.g., ligaments, tendons and the like) to bone and bone-like structures.

## Claims

1. An apparatus for attaching soft tissues to bone of the type comprising a surgical fastener (100) adapted to directly engage a hole in the bone which includes a shank (105) having a distal end, a proximal end, a longitudinal axis and at least one projection extending outwardly of the shank intermediate the distal and proximal ends thereof, and an enlarged head (110) coupled to the proximal end of the shank (105), characterised in that:
(1) the distal end of the shank (105) has a frustoconical configuration; and
(2) the at least one projection comprises at least two discrete, substantially identical ribs (135), each rib (a) completely encircling the shank (105) transversely to the longitudinal axis, (b) being longitudinally spaced from adjacent ones of said at least two ribs (135) so as to promote bone ingrowth between adjacent ones of the ribs (135), (c) including a first longitudinal portion (140) and a second longitudinal portion (145), the first longitudinal portion (140) being disposed toward the distal end of the shank (105), the second longitudinal portion (145) being disposed toward the proximal end of the shank (105) and the first longitudinal portion (140) having a smaller circumference than the second longitudinal portion (145), (d) a bore (165) extending completely through the shank and the head along the longitudinal axis, and (e) delivery means for delivering the fastener to the attachment point of the fastener to the bone.

2. An apparatus as claimed in claim 1, characterised in that the second portion (145) of each of the ribs (135) has an outer surface which extends parallel to the longitudinal axis of the shank (105).

3. An apparatus as claimed in claim 1 or 2, characterised in that the second portion (145) of each of the ribs (135) has an outer surface which extends parallel to the longitudinal axis of the shank (105) and along about half of the length of the rib (105) as measured along the longitudinal axis.

4. An apparatus as claimed in any preceding claim, characterised in that each of the ribs (135) is spaced from adjacent ones of said at least two ribs (135) by a distance greater than the length of one of the ribs as measured along the longitudinal axis of the shank (105).

5. An apparatus as claimed in any preceding claim, characterised in that the portion of the shank (105) disposed between the proximalmost of the at least two ribs (135) and the proximal end of the shank (105) has a diameter greater than the portion of the shank (105) disposed between the distalmost one of the at least two ribs (135) and the distal end of the shank (105).

6. An apparatus as claimed in any preceding claim, characterised in that a fillet is attached to and extends between the head (110) and the shank (105).

7. An apparatus as claimed in any preceding claim, characterised in that the portion of the shank (105) disposed between adjacent ones of the at least two ribs (135) has a diameter which is (a) smaller than the diameter of the shank (105) between the proximalmost one of the at least two ribs (135) and the proximal end of the shank (105) and (b) larger than the diameter of the portion of the shank (105) located between the distalmost one of the at least two ribs (135) and the distal end of the shank (105).

8. An apparatus as claimed in any preceding claim, characterised in that each rib (155) has a proximally facing end which is undercut.

9. An apparatus as claimed in any preceding claim, characterised in that the enlarged head (110A) comprises at least one projection (170A) extending away from the head (110A) toward the distal end of the shank (115A), the at least one projection (170A) being radially spaced from the shank (115A).

10. An apparatus as claimed in any preceding claim, characterised in that the bone has an outer cortical or hard region and an inner cancellous or soft region, and the longitudinal distance between the enlarged head and the proximalmost one of the at least two ribs is greater than the combined width of the object and the cortical or hard region of the bone, such that upon engagement of the fastener with the bone, the head lies against the object and the proximalmost one of the at least two ribs lies within the cancellous or soft region of the bone.

11. An apparatus as claimed in any preceding claim, characterised in that the delivery means includes a guide wire (400) having first and second ends and being adapted to have its first end disposed adjacent to the attachment point of the fastener to the bone; and the bore and the guide wire being sized relative to one another such that the guide wire may be slidingly inserted through the bore.

12. An apparatus as claimed in claim 11, characterised in that the apparatus further comprises hole forming means for forming a hole in the bone when the object is positioned against the bone, the hole forming means comprising a cannulated drilling member (500) having an axial bore adapted to accommodate the guide wire (400) and sized relative to the fastener such that the fastener may be snugly received within the hole formed in the bone by the hole forming means.

## Patentansprüche

1. Vorrichtung zum Befestigen weicher Gewebe an Knochen, vom Typ mit einem chirurgischen Befestigungsmittel (100), das so ausgebildet ist, daß es direkt in ein Loch in einem Knochen eingreift und einen Schaft (105) mit einem körperfernen Ende, einem körpernahen Ende und mit einer Längsachse und mit mindestens einem Vorsprung aufweist, der sich vom Schaft zwischen dessen körperfernem und dessen körpernahem Ende erstreckt, und einem vergrößerten Kopf (110), der mit dem körpernahen Ende des Schafts (105) verbunden ist, **dadurch gekennzeichnet**, daß:
(1) das körperferne Ende des Schafts (105) kegelstumpfförmige Konfiguration aufweist; und
(2) der mindestens eine Vorsprung mindestens zwei einzelne, im wesentlichen identische Rippen (135) aufweist, von denen jede Rippe
(a) den Schaft (105) quer zur Längsachse vollständig umschließt,
(b) in Längsrichtung von den benachbarten mindestens zwei Rippen (135) beabstandet ist, um das Knocheneinwachsen zwischen benachbarten Rippen (135) zu fördern,
(c) einen ersten Längsabschnitt (140) und einen zweiten Längsabschnitt (145) aufweist, wobei der erste Längsabschnitt (140) zum körperfernen Ende des Schafts (105) hin angeordnet ist, der zweite Längsabschnitt (145) zum körpernahen Ende des Schafts (105) hin angeordnet ist und der erste Längsabschnitt (140) einen kleineren Umfang als der zweite Längsabschnitt (145) aufweist,
(d) sich eine Bohrung (165) vollständig durch den Schaft und den Kopf entlang der Längsachse erstreckt, und
(e) eine Zuführeinrichtung zum Zuführen des Befestigungsmittels zum Befestigungspunkt für das Befestigungsmittel am Knochen.

2. Vorrichtung nach Anspruch 1, **dadurch gekennzeichnet,** daß der zweite Abschnitt (145) jeder der Rippen (135) eine Außenfläche aufweist, die sich parallel zur Längsachse des Schafts (105) erstreckt.

3. Vorrichtung nach einem der Ansprüche 1 oder 2, **dadurch gekennzeichnet**, daß der zweite Abschnitt (145) jeder der Rippen (135) eine Außenfläche aufweist, die sich parallel zur Längsachse des Schafts (105) und ungefähr über die Hälfte der Länge der Rippe (135) entlang der Längsachse erstreckt.

4. Vorrichtung nach einem der vorstehenden Ansprüche, **dadurch gekennzeichnet**, daß jede der Rippen (135) von benachbarten mindestens zwei Rippen (135) um einen Abstand beabstandet ist, der größer ist als die Länge einer der Rippen gemessen entlang der Längsachse des Schafts (105).

5. Vorrichtung nach einem der vorstehenden Ansprüche, **dadurch gekennzeichnet**, daß der Abschnitt des Schafts (105), der zwischen den dem körpernahen Ende am nächsten liegenden mindestens zwei Rippen (135) und dem körpernahen Ende des Schafts (105) liegt, einen größeren Durchmesser als derjenige Abschnitt des Schafts (105) aufweist, der zwischen der dem körperfernen Ende nächstliegenden der zwei Rippen (135) und dem körperfernen Ende des Schaftes (105) liegt.

6. Vorrichtung nach einem der vorstehenden Ansprüche, **dadurch gekennzeichnet**, daß eine Ausrundung angebracht ist, die sich zwischen dem Kopf (110) und dem Schaft (105) erstreckt.

7. Vorrichtung nach einem der vorstehenden Ansprüche, **dadurch gekennzeichnet**, daß der zwischen benachbarten mindestens zwei Rippen (135) angeordnete Abschnitt des Schafts (105) einen Durchmesser aufweist, der (a) kleiner ist als der Durchmesser des Schafts (105) zwischen der dem körpernahen Ende naheliegenden der mindestens zwei Rippen (135) und dem körpernahen Ende des Schafts (105), und (b) größer ist als der Durchmesser des Abschnitts des Schafts (105) zwischen der dem körperfernen Ende nächstliegenden der zwei Rippen (135) und dem körperfernen Ende des Schafts (105).

8. Vorrichtung nach einem der vorstehenden Ansprüche, **dadurch gekennzeichnet**, daß jede Rippe (155) ein zum körpernahen Ende zeigendes Ende aufweist, das hinterschnitten ist.

9. Vorrichtung nach einem der vorstehenden Ansprüche, **dadurch gekennzeichnet**, daß der vergrößerte Kopf (110A) mindestens einen Vorsprung (170A) aufweist, der vom Kopf (110A) weg zum körperfernen Ende des Schafts (115A) zeigt, wobei der mindestens eine Vorsprung (170A) radial vom Schaft (115A) beabstandet ist.

10. Vorrichtung nach einem der vorstehenden Ansprüche, **dadurch gekennzeichnet**, daß der Knochen einen äußeren kortikalen oder harten Bereich und einen inneren schwammigen oder weichen Bereich aufweist und daß der Längsabstand zwischen dem vergrößerten Kopf und der dem körpernahen Ende nächstliegenden der mindestens zwei Rippen größer ist als die kombinierte Breite aus dem Gegenstand und dem kortikalen oder harten Bereich des Knochens, so daß beim Angriff des Befestigungsmittels am Knochen der Kopf am Gegenstand anliegt und die dem körpernahen Ende nächstliegende der zwei Rippen innerhalb des schwammigen oder weichen Bereichs des Knochens liegt.

11. Vorrichtung nach einem der vorstehenden Ansprüche, **dadurch gekennzeichnet**, daß die Zuführeinrichtung einen Führungsdraht (400) mit einem ersten und einem zweiten Ende aufweist, der so ausgebildet ist, daß sein erstes Ende angrenzend an den Befestigungspunkt des Befestigungsmittels am Knochen liegt; und die Bohrung und der Führungsdraht relativ zueinander so bemessen sind, daß der Führungsdraht gleitend durch die Bohrung eingeführt werden kann.

12. Vorrichtung nach Anspruch 11, **dadurch gekennzeichnet**, daß sie ferner eine Lochherstellungseinrichtung zum Herstellen eines Lochs im Knochen aufweist, wenn der Gegenstand gegen den Knochen liegend positioniert wird, wobei die Lochherstellungseinrichtung ein hohles Bohrerteil (500) mit einer axialen Bohrung aufweist, die so ausgebildet ist, daß sie zum Führungsdraht (400) paßt und in bezug auf das Befestigungsmittel so bemessen ist, daß das Befestigungsmittel satt sitzend im Loch aufgenommen werden kann, das im Knochen durch die Lochherstellungseinrichtung ausgebildet wurde.

## Revendications

1. Appareil destiné à fixer des tissus mous sur un os du type comportant un élément de fixation chirurgical (100) prévu pour engager directement un trou dans l'os, qui comprend une tige (105) ayant une extrémité distale, une extrémité proximale, un axe longitudinal et au moins une saillie s'étendant vers l'extérieur de la tige entre les extrémités distale et proximale de celle-ci, et une tête agrandie (110) reliée à l'extrémité proximale de la tige (105), caractérisé en ce que :
(1) l'extrémité distale de la tige (105) a une configuration tronconique; et
(2) la saillie comprend au moins deux nervures séparées sensiblement identiques (135), chaque nervure (a) entourant totalement la tige (105) transversalement à l'axe longitudinal, (b) étant longitudinalement espacée des nervures adjacentes (135) de façon à faciliter la croissance de l'os entre les nervures adjacentes (135), (c) comprenant une première partie longitudinale (140) et une deuxième partie longitudinale (145), la première partie longitudinale (140) étant disposée en direction de l'extrémité distale de la tige (105), la deuxième partie longitudinale (145) étant disposée en direction de l'extrémité proximale de la tige (105) et la première partie longitudinale (140) ayant une circonférence plus faible que la deuxième partie longitudinale (145), (d) un alésage (165) s'étendant complètement à travers la tige et la tête le long de l'axe longitudinal, et (e) des moyens de mise en place destinés à amener l'élément de fixation vers le point de fixation de l'élément de fixation sur l'os.

2. Appareil selon la revendication 1, caractérisé en ce que la deuxième partie (145) de chacune des nervures (135) a une surface extérieure qui s'étend parallèlement à l'axe longitudinal de la tige (105).

3. Appareil selon la revendication 1 ou 2, caractérisé en ce que la deuxième partie (145) de chacune des nervures (135) a une surface extérieure qui s'étend parallèlement à l'axe longitudinal de la tige (105) et sur environ la moitié de la longueur de la nervure (135) telle que mesurée le long de l'axe longitudinal.

4. Appareil selon l'une quelconque des revendications précédentes, caractérisé en ce que chacune des nervures (135) est espacée des nervures adjacentes (135) d'une distance supérieure à la longueur de l'une des nervures telle que mesurée le long de l'axe longitudinal de la tige (105).

5. Appareil selon l'une quelconque des revendications précédentes, caractérisé en ce que la partie de la tige (105) disposée entre la nervure la plus proximale et l'extrémité proximale de la tige (105) a un diamètre supérieur à la partie de la tige (105) disposée entre la nervure la plus distale et l'extrémité distale de la tige (105).

6. Appareil selon l'une quelconque des revendications précédentes, caractérisé en ce qu'un congé est fixé à et s'étend entre la tête (110) et la tige (105).

7. Appareil selon l'une quelconque des revendications précédentes, caractérisé en ce que la partie de la tige (105) disposée entre les nervures adjacentes (135) a un diamètre qui est (a) plus faible que le diamètre de la tige (105) entre la nervure (135) la plus proximale et l'extrémité proximale de la tige (105) et (b) plus grand que le diamètre de la partie de la tige (105) disposée entre la nervure (135) la plus distale et l'extrémité distale de la tige (105).

8. Appareil selon l'une quelconque des revendications précédentes, caractérisé en ce que chaque nervure (135) a une extrémité dirigée de manière proximale qui est en contre-dépouille.

9. Appareil selon l'une quelconque des revendications précédentes, caractérisé en ce que la tête agrandie (110A) comporte au moins une saillie (170A) s'étendant à l'écart de la tête (110A) en direction de l'extrémité distale de la tige (115A), la saillie (170A) étant espacée radialement de la tige (115A).

10. Appareil selon l'une quelconque des revendications précédentes, caractérisé en ce que l'os présente une zone dure ou corticale extérieure et une zone poreuse ou molle intérieure, et la distance longitudinale entre la tête agrandie et la nervure la plus proximale est supérieure à la largeur combinée de l'objet et de la zone dure ou corticale de l'os, de sorte que, lors de l'engagement de l'élément de fixation avec l'os, la tête s'étend contre l'objet et la nervure la plus proximale s'étend à l'intérieur de la zone poreuse ou molle de l'os.

11. Appareil selon l'une quelconque des revendications précédentes, caractérisé en ce que les moyens de mise en place comprennent un fil de guidage (400) ayant des première et deuxième extrémités et qui est prévu pour avoir sa première extrémité disposée de façon adjacente au point de fixation de l'élément de fixation sur l'os; et l'alésage et le fil de guidage sont dimensionnés l'un par rapport à l'autre de telle sorte que le fil de guidage peut être inséré de façon coulissante dans l'alésage.

12. Appareil selon la revendication 11, caractérisé en ce que l'appareil comporte en outre des moyens de formation de trou destinés à former un trou dans l'os lorsque l'objet est positionné contre l'os, les moyens de formation de trou comportant un élément de forage creux (500) ayant un alésage axial prévu pour recevoir le fil de guidage (400) et dimensionné par rapport à l'élément de fixation de telle sorte que l'élément de fixation peut être intimement reçu dans le trou formé dans l'os par les moyens de formation de trou.
